# EUROPEAN PATENT SPECIFICATION

(11) **EP 1 707 110 B1**
(45) Date of publication and mention of the grant of the patent: **22.10.2008**
(21) Application number: 06111963.2
(22) Date of filing: 30.03.2006
(51) Int. Cl.: A61B 5/00

(54) **Apparatus for detecting cardiac signals**
Vorrichtung zur Detektion von Herzsignalen
Appareil pour détecter des signaux cardiaques

(30) Priority: 31.03.2005 IT MI20050523
(43) Date of publication of application: 04.10.2006
(73) Proprietor: Q & S S.R.L., 42100 Reggio Emilia (IT)
(72) Inventor: Quaini, Giuseppe, 42100, Reggio Emilia (IT)
(74) Representative: Baroni, Matteo

(56) References cited:
- WO-A-01/85019
- US-A- 5 303 702
- US-A- 5 749 367
- US-A1- 2004 167 416

## Description

The present invention relates to an apparatus for detecting cardiac signals.

It is known that it is possible to evaluate the conditions of correct operation in the cardiac system of an individual through different types of tests; said tests can be also carried out with reference to signals of the electric type generated by the heart during the ventricles' contraction and relaxation movements.

A first check that can be carried out is the so-called static electrocardiogram (ECG); through it there are recorded the action potentials of the myocardial fibres detected through suitable electrodes put into contact with the patient's skin, the patient being in a rest position.

Usually ten electrodes are employed, so as to be able to detect the electric-axis projections of the heart in different planes; the registration can last some ten seconds and, after the detecting step has been completed, the ECG traces are submitted to the physician's attention, whose task is to make a diagnosis.

A second test that is usually carried out is the so-called dynamic electrocardiogram according to Holter (or more simply "Holter") which consists in recording the electrocardiographic signals over a period of time until 24 hours; due to the high amount of data to be taken into account, in this case only four electrodes can be used, which are addressed to detecting some projections of the electric axis that are representative of the heart's behaviour.

The registration can be carried out either on a magnetic medium (such as a common cassette of music), or on an electronic medium (such as a memory card).

When the detecting step has been completed, in this case too the whole ECG traces must be examined by the competent physician so that arising of possible pathologies can be recognised.

In the light of the above it is apparent that, above all in the case of tests of the Holter type, the data amount to be analysed is very huge and the physician's task is particularly long and difficult.

To partly remedy this drawback, in the known art apparatus known as "ECG Event Recorders" are made available; they consist of devices set to record the electrocardiographic signal following an activation command entered by the user.

In other words, an ECG Event Recorder is usually in a stand-by condition; when the patient feels symptoms of bad operation of his/her cardiac system, he/she presses a suitable push-button (or similar activation instrument) so as to start recording of the cardiac signal.

Consequently, a registration concerning the periods of time during which the patient has felt symptoms of bodily discomfort are supplied to the physician to enable him/her to correctly interpret the related ECG traces.

It is apparent that the last-mentioned solution too has clear operating drawbacks; first of all possible pathologies that do not cause well-apparent and clearly identifiable symptoms to the patient cannot be taken into account.

In addition, even if the patient is able to activate the device on arising of the symptom, it is not possible to check the cardiac system in the time interval immediately preceding starting of the registration; this check however can be very useful to enable best understanding of the occurred trouble or disease.

Document US 5749367 discloses a heart monitoring apparatus and method, wherein an electrocardiograph signal is obtained from a patient and processed to enhance the salient features and to suppress noise. A plurality n of values representative of the features of the electrocardiograph signal are generated and used in a Kohonen neural network to generate an n dimensional vector. This vector is compared with a stored plurality m of n dimensional reference vectors defining an n dimensional Kohonen feature map to determine the proximity of the vector to the reference vectors. If it is determined by the Kohonen neural network that the vector is within or beyond a threshold range of the reference vectors a signal is output which can be used to initiate an event such as the generation of an alarm or the storage of ECG data.

Document US200470167416 discloses a minimally invasive, implantable monitor and associated method for chronically monitoring a patient's hemodynamic function based on signals sensed by one or more acoustical sensors. The monitor may be implanted subcutaneously or submuscularly in relation to the heart to allow acoustic signals generated by heart or blood motion to be received by a passive or active acoustical sensor. Circuitry for filtering and amplifying and digitizing acoustical data is included, and sampled data may be continuously or intermittently written to a looping memory buffer. ECG electrodes and associated circuitry may be included to simultaneously record ECG data. Upon a manual or automatic trigger event acoustical and ECG data may be stored in long-term memory for future uploading to an external device. The external device may present acoustical data visually and acoustically with associated ECG data to allow interpretation of both electrical and mechanical heart function.

It is an aim of the present invention to make available an apparatus for detecting cardiac signals that is able to detect important cardiac-signal portions, i.e. portions associated with arising of real pathologies in the patient in a reliable manner.

Another aim of the invention is to provide an apparatus enabling an immediate comparative analysis between the moment the symptoms are felt by the patient and the real diseases of the cardiac system.

A further aim of the invention is to make available an apparatus capable of operating in a complete and reliable manner while using a memory of restricted capacity.

The foregoing and further aims are substantially achieved by an apparatus for detecting cardiac signals in accordance with the features recited in the appended claims.

Further features and advantages will become more apparent from the detailed description of a preferred but not exclusive embodiment of the apparatus in accordance with the invention.

This description will be provided with reference to the accompanying drawings, given by way of non-limiting example, in which:
- Fig. 1 diagrammatically shows the apparatus of the invention applied to a patient;
- Fig. 2 shows a block diagram of the apparatus seen in Fig. 1;
- Fig. 3 diagrammatically shows a signal detected by the apparatus in Fig. 1;
- Fig. 4 shows a diagram representing different portions of the signal seen in Fig. 3.

With reference to the drawings, an apparatus for detecting cardiac signals in accordance with the present invention has been generally identified with reference numeral 1.

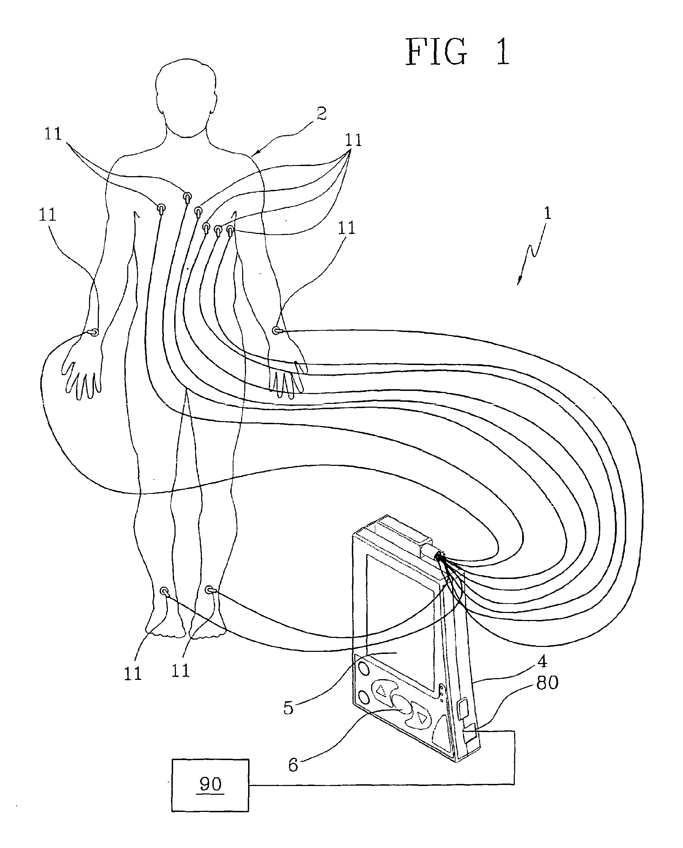

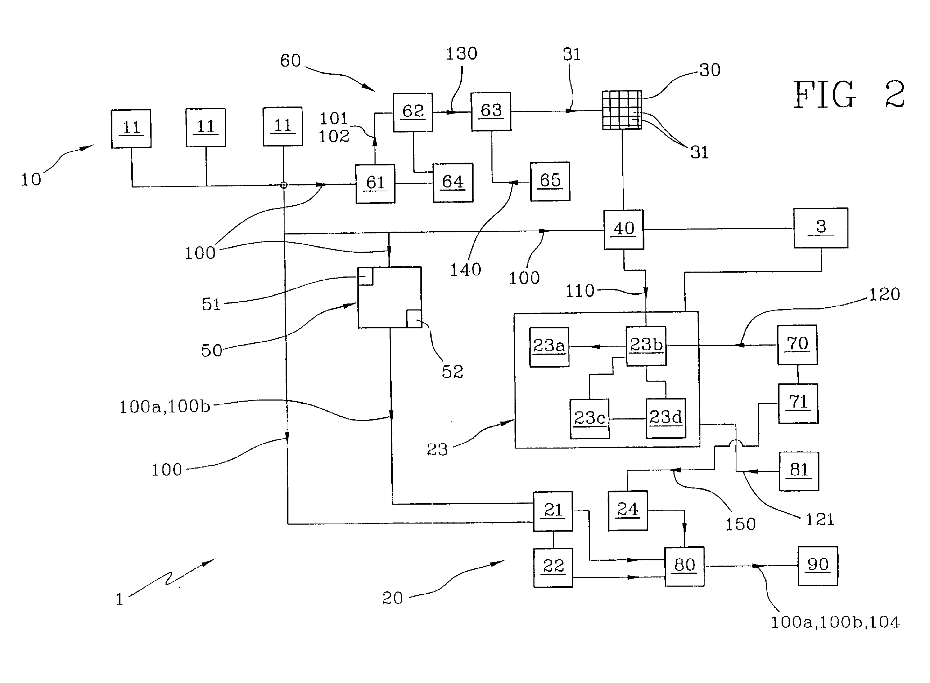

Apparatus 1 (Figs. 1 and 2) first of all comprises sensor means 10 to detect at least one cardiac signal 100 representing operation of the heart in a patient 2.

The sensor means 10 preferably comprises a plurality of electrodes 11 that are put into contact with the patient's skin when apparatus 1 is in use conditions.

The electrodes 11 can be conveniently associated with adhesive means or means consisting of suction cups (not shown in the figure), to ensure correct and fixed positioning of the electrodes 11 on the patient's body.

The sensor means 10 is positioned in a manner adapted to detect the signal 100 representative of the electric potentials generated in the heart. For carrying out a detection of the "static" electrocardiographic type, the electrodes 11 are positioned on the thorax (6 electrodes) and the limbs of the patient 2 (one electrode for each limb). For carrying out a detection of the Holter type (or dynamic electrocardiogram) the electrodes 11 are located on the patient's thorax (4 electrodes).

Advantageously, the electrodes 11 of apparatus 1 are positioned at each of the above stated locations so that it is possible to carry out a detection both in the case of a static electrocardiogram and in the case of a dynamic electrocardiogram.

For storage of the thus detected information, apparatus 1 further comprises a storage unit 20; the latter is set to store the cardiac signal 100 at least partly. In more detail, the storage unit 20 is provided with a permanent memory 21 into which a predetermined number of portions of the cardiac signal 100 is stored.

In the present context the term "permanent" means that the data stored in memory 21 are maintained until an express erase command entered by an operator is received; in particular, the term "permanent" is herein used in opposition to the wording "temporary" that, as better clarified in the following, refers to a buffer in which data is continuously overwritten, so that in this way the less recent information is lost.

Storage of the cardiac signal 11 in the permanent memory 21 can be activated depending on the characteristics of the cardiac signal 100 itself and on main reference parameters 31 relating to said cardiac signal.

In fact, apparatus 1 comprises an auxiliary memory 30, into which the main reference parameters 31 are stored; the main reference parameters 31 are representative of predetermined cardiac pathologies that can arise in the patient 2.

These parameters are preferably defined by recognised Bodies on an international scale so that apparatus 1 can meet the required quality and reliability standards.

Apparatus 1 further comprises a comparator block 40 connected to the sensor means 10 and the auxiliary memory 30; the comparator block 40 compares the cardiac signal 100 and the main reference parameters 31 with each other and depending on this comparison, if necessary, generates a suitable main command signal 110 to activate storage of the cardiac signal 100 into the permanent memory 21.

In this way, when an anomaly (i.e. one of the pathologies previously stored by means of the main reference parameters 31), is detected in the cardiac signal 100, storage of a portion of the cardiac signal 100 itself is started, so that an analysis of said signal can be subsequently carried out by a competent physician.

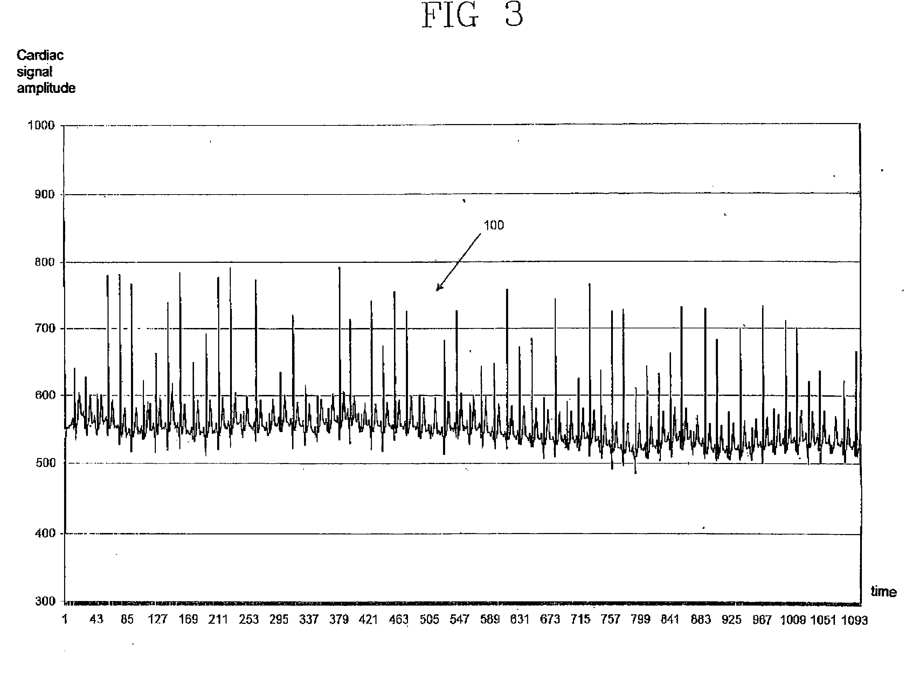

By way of example, the anomaly represented in Fig. 3 consists of a non-uniform time distribution of the different peaks present in the ECG traces (arrhythmia).

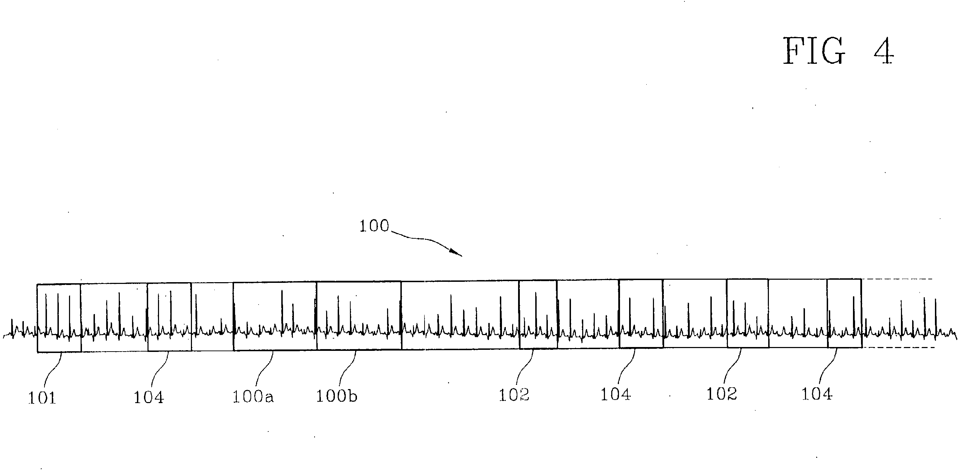

On the contrary, diagrammatically shown in Fig. 4 are the different portions of the cardiac signal 100 that are detected and stored; these portions follow each other, by way of example from left to right.

As mentioned above, the main reference parameters 31 are representative of pathologies that can arise in the patient's cardiac system.

To make the decision taken by the comparator block 40 reliable, it is necessary for the main reference parameters 31 to be suitably adjusted depending on the general physical characteristics of the patient 2. In fact, individuals of different sex, height, weight, age, etc. have different discrimination thresholds for identification of the cardiac pathologies.

Therefore, apparatus 1 further comprises a learning unit 60 provided with a learning register 61 into which at least one initial portion 101 of the cardiac signal 100 is stored; practically, when apparatus 1 starts operating, the first portion of the cardiac signal that has been detected (the 8 first seconds, for example) is stored into the learning register. Then, suitable processing means 62 carries out calculation of the input parameters 130 depending on the characteristics of this initial portion 101; the input parameters 130 are representative of the main characteristics of the cardiac signal 100 under conditions of correct operation of the patient's organism.

The learning unit 60 further comprises an auxiliary register 65 into which general parameters 140 representative of cardiac pathologies are stored; these parameters 140 are exactly of general type and do not take the patient's specific characteristics into account.

The learning unit 60 further comprises a calculation block 63 to calculate the main reference parameters 31 depending on said input parameters 130; in particular, the calculation block 63 is connected to the processing means 62 and the auxiliary register 65 to define the main reference parameters 31 depending both on the input parameters 130 (i.e. those concerning the patient's characteristics), and on the general parameters 140 (representative of the pathologies that may be detected). The main reference parameters 31 thus calculated are then stored into the auxiliary memory 30.

For the purpose of making determination of the main reference parameters 31 more precise, the learning unit 60 further comprises updating means 64; this means is connected to the learning register 61 to activate storage of auxiliary portions 102 of the cardiac signal 100 as well.

Said auxiliary portions 102 are preferably detected and stored at predetermined time intervals, according to a pre-established frequency; by way of example, the auxiliary portions 102 can last about 30 seconds and be detected every three hours.

The updating means 64 is also connected to the processing means 62 to activate said processing means and update the input parameters 130 depending on the characteristics of the auxiliary portions 102 of the cardiac signal 100.

Then also the calculation block 63 is activated so as to enable determination of the main reference parameters 31 also taking into account the input parameters 130 updated on the basis of the characteristics of the auxiliary portions 102.

In the light of the above, it is apparent that the reference parameters 31 can be defined in an optimal manner so as to make operation of the comparator block 40 and the whole apparatus 1 in general, precise and reliable.

In the following of the present specification, the modalities for storage of the cardiac signal 100 will be described.

Apparatus 1 comprises a temporary storage register 50, connected to the sensor means 10 to temporarily store portions of the cardiac signal 100 of a predetermined time duration.

As better clarified in the following, depending on said main command signal 110, the contents of the temporary register 50 are transferred to the memory 21 for permanent storage.

Practically, the temporary register 50 can be a "circular" buffer, i.e. a buffer in which data are sequentially stored and overwritten according to a predetermined frequency. In more detail, an initial memory address 51 and a final memory address 52 define the limits of the temporary register 50; in other words, the memory cells of the register 50 are arranged according to a predetermined sequence and the initial address 51 and final address 52 identify the first one and last one respectively of said cells.

Data storage into the temporary register 50 therefore follows the predetermined arrangement of said cells.

In particular, storage of the cardiac signal 100 takes place sequentially into the temporary register 50, from the initial address 51 to the final address 52.

It is to be pointed out that the temporary register 50 has a storage capacity that is not sufficient to hold the whole cardiac signal 100; for instance, the cardiac signal 100 can have a time duration of 24 hours (in the case of an analysis of the Holter type), while the temporary register 50 has a capacity sufficient to hold about a 40 seconds' registration.

On reaching of the final address 52, i.e. when the temporary register 50 is completely filled, storage goes on through overwriting of the contents of the register 50 itself, starting from the initial address 51. In this way, the less recent data is replaced by the just detected one; therefore the 40 last seconds of the cardiac-signal detection will be always available.

The temporary storage of the cardiac signal 100 in the temporary register 50 takes place as above described, irrespective of the generation of the command signal 110; the last-mentioned signal is used to at least partly transfer the contents of the temporary register 50 to the permanent memory 21.

In fact, when following the comparison between the cardiac signal 100 and the main reference parameters 21 the comparator block 40 activates the storage unit 20, said storage unit stores a first and a second portion 100a, 100b of the cardiac signal 100 into its permanent memory 21.

In particular, the second portion 100b is the cardiac-signal portion immediately preceding the instant at which the storage unit 20 is activated; this second portion 100b is fetched from the temporary register 50 that, as above said, keeps all data present therein until the same are overwritten. For instance, the second portion 100b can have a duration of about 20 seconds.

The first portion 100a on the contrary is the cardiac-signal portion immediately following activation of the storage unit 20; the first portion100a can therefore be stored into the permanent memory 21 in real time, directly coming from the sensor means 10 or passing through the temporary register 50.

The first portion 100a too can, by way of example, have a duration of about 20 seconds; the result of the above described procedure is that, following generation of the main command signal 110, an assembly of data representing the cardiac signal 100 during the 20 seconds preceding and the 20 seconds following detection of the anomaly can be stored into the permanent memory 21.

Obviously the above stated time lengths are given by way of example and can be varied depending on specific requirements without being prejudicial to the general character of the discussed matter.

Advantageously, the storage unit 20 is provided with a time storage block 22 to store data representing the time instants during which the cardiac signal 100 is detected and/or stored into said permanent memory 21.

In this way, the physician that will be entrusted with the task of analysing the registered information is able to understand when during the day the anomaly that caused partial storage of the cardiac signal 100 has occurred.

In the preferred embodiment, apparatus 1 further comprises a driving block 70 that can be activated by the patient 2 when the latter feels troubles that can be caused by a bad operation of the cardiac system.

The driving block 70 in this case generates an auxiliary command signal 120 to activate the storage unit 20 and obtain partial storage of the cardiac signal 100 following the same modalities as those described with reference to the main command signal 100.

In more detail, even in case of activation by the patient 2, a first and a second portions 100a, 100b of the cardiac signal 100 will be stored in the permanent memory 21, which portions respectively relate to the time intervals immediately following and immediately preceding reception of the auxiliary command signal 120 by the storage unit 20.

In addition, the time storage block 22 carries out data storage to identify the instant at which the auxiliary command signal 120 was generated, i.e. the moment at which the patient 2 felt troubles.

Practically, the driving block 70 can consist of a push-button or equivalent device capable of generating the auxiliary command signal 120.

It is possible for the main command signal 110 or the auxiliary command signal 120 to be received by the storage unit 20 when storage of the cardiac signal 100 into the permanent memory 21 is already taking place; typically this can occur when a given pathology is first detected by apparatus 1 and, shortly afterwards, due to the generated trouble, it is also felt by the patient. In this case overlapping of the two commands must be suitably controlled.

To this aim the storage unit 20 comprises a verification block 23 connected to the comparator block 40 and the driving block 70 to receive the main command signal 110 and auxiliary command signal 120. The verification block 23 is provided with control means 23a to verify whether, on reception of the main command signal 110 or the auxiliary command signal 120, storage of the cardiac signal 100 into the permanent memory 21 is already active or not. If it is not so, drive means 23c activates said storage so that the cardiac signal 100 is recorded following the above described modalities.

If, on the contrary, storage is already active, since it does not need to be activated, timing means 23b stores data representing the instant at which the command signal 110, 120 was received; in addition, a time correction block 23d has the task of extending the time length of the first portion 100a of the cardiac signal.

In more detail, let us assume, by way of example, that first a main command signal 110 and then an auxiliary command signal 120 are received in succession. Following reception of the main command signal 110, the storage unit 20 is activated to store said first portion 100a of the cardiac signal 100, which first portion has a predetermined duration (as stated above, of 20 seconds for example).

If, meanwhile, an auxiliary command signal 120 is received, the time correction block 23d causes the first portion 100a of the cardiac signal 100 not to finish after 20 seconds from reception of the main command signal, but to go on until expiry of 20 seconds starting from reception of the auxiliary command signal 120.

Obviously the above stated 20 seconds' time interval is given just as an indication and can be varied depending on the specific requirements.

In addition, it is provided that, following reception of the main command signal 110 and the auxiliary command signal 120 different time lengths can be used, and respectively a first time length and a second time length that are different from each other.

In addition to said auxiliary command signal 120, the patient 2 is allowed to enter auxiliary data 150 through suitable auxiliary-data input means 71; this auxiliary data can refer to the type of symptoms felt by the patent 2 when he/she activates the driving block 70 to generate the auxiliary command signal 120.

For instance, the auxiliary-data input means 71 can be obtained by displaying a menu on the display of apparatus 1 on which different choices are shown (weariness sensation, sudden increase in the heart rate, etc.) among which the patient 2 can select the right one; in this manner the analysis and subsequent diagnosis by the physician that will examine the detected data are facilitated.

Advantageously, the auxiliary data 150 is stored into a secondary register 24 of the storage unit 20 so that the physician will have availability of them when necessary.

In addition to the above, apparatus 1 can be also provided with auxiliary activation means 81 to activate a permanent storage of secondary portions 104 of the cardiac signal 100 at predetermined time intervals, irrespective of the main 110 or auxiliary 120 command signals. In other words, the auxiliary activation means 81 is connected to the storage unit 20 to store secondary portions 104 of the cardiac signal 100 into the permanent memory 21, which secondary portions are selected randomly during application of apparatus 1.

For instance, each secondary portion 104 can have a duration of about 30 seconds and can be detected after a 3 hours' interval from the preceding secondary portion.

Storage of the secondary portions 104 aims at giving the physician a general picture of the patient's cardiac system operation in the space of the 24 hours during which apparatus 1 is used, irrespective of the anomalies detected by the comparator block 40 and/or the sensations of bodily discomfort felt by the patient.

Preferably, the auxiliary activation means 81, when a secondary portion 104 must be detected and stored, transmit an additional command signal 121 to the verification block 23.

If on occurrence of this situation the storage unit 20 is not active, the same is activated for storage of the secondary portion 104. Vice versa, if the storage unit 20 has already been activated previously (following a main 110 or auxiliary 120 command signal), the time correction block 23d, if necessary, carries out extension of the time interval length during which the storage unit keeps active, so that the storage unit 20 itself is deactivated at the end of the secondary portion 104.

Apparatus 1 may further comprise a communication interface 80 for a connection with a remote processing unit 90, of the telephone type for example; said processing unit can practically be a computer capable of receiving the different detecting operations carried out by the apparatus 1 itself. In particular, the interface 80 can allow a connection to a telephone or telecommunication network, through which apparatus 1 can transmit the detected portions of the cardiac signal 100 to the physician entrusted with the patient's care, taking the due precautions in terms of security and privacy; these transmitted portions can comprise the first and second portions 100a, 100b and/or the secondary portions 104.

Preferably, the remote computer 90 is such programmed that it can decode the signal transmitted by the apparatus 1, store it into a suitable database and dispose the same on an electronic graph paper sheet for medical report.

Through suitable alert systems (e-mail message, SMP, etc.) the physician making the report is informed about the fact that said transmission has occurred and that the signal is available for medical report.

Through suitable algorithms of the digital type (using a smart card, for example) it is possible to give the contents of said communications a medico-legal value, so that they can fully replace the usual paper documents.

In the light of the above, the apparatus 1 is provided with an electronic processing system (a microprocessor for example) capable of performing the described functions; in other words, said comparator block 40, storage unit 20, learning unit 60 can be formed with a single suitably programmed CPU.

The division into the different blocks has the only purpose of clarifying which are the functions performed by the apparatus 1 in accordance with the invention.

Likewise, the permanent memory 21, auxiliary memory 30, temporary register 50, secondary register 24, auxiliary register 65 and time storage block 22 can be formed with one or more memories of the electronic type associated with the above mentioned CPU.

It is also to be noted that apparatus 1 is provided with a digital reference clock (not shown in the figures) at least connected with the time storage block 22 and the timing means 23b in order to enable availability of the time reference for storage of all time data relating to the detecting operations carried out.

Apparatus 1 is further conveniently provided with a power unit 3, preferably of the rechargeable type, connected to the different blocks described above; in this manner apparatus 1 is very practical and of easy use.

As diagrammatically shown in Fig. 1, apparatus 1 is provided with a housing 4 within which all circuitry necessary to perform the above described functions is housed.

In particular, formed in said housing is the display 5 through which the user can display the detected and stored data and a keyboard 6 enabling him/her to interact with the apparatus 1 itself.

The invention achieves important advantages.

First of all, the apparatus in accordance with the invention is able to detect important portions of the cardiac signal, i.e. portions associated with arising of real pathologies in the patient, in a reliable manner.

In addition the competent physician is allowed to perform an immediate comparative analysis between the symptoms felt by the patient and the true troubles present in the cardiac system.

Another advantage is found in the fact that the apparatus, for operating in a correct and complete manner, only requires a memory of a very limited capacity, since only the important and significant information is detected.

## Claims

1. An apparatus for detecting cardiac signals, comprising:
- sensor means (10) to detect at least one cardiac signal (100) of a patient (2);
- a storage unit (20) connected to said sensor means (10) to store said cardiac signal (100) at least partly,
- an auxiliary memory (30) containing one or more main reference parameters (31) for said cardiac signal (100);
- a comparator block (40) to compare said cardiac signal (100) and said one or more main reference parameters (31) with each other and activate said storage unit (20) depending on said comparison through a related main command signal (110),
- a driving block (70) to be operated by the patient (2) to activate said storage unit (20) through an auxiliary command signal (120);
wherein said storage unit (20) comprises a verification block (23) connected to said driving block (70) and said comparator block (40) to receive said main command signal (110) and said auxiliary command signal (120) and activate storage of said cardiac signal (100) depending on said main (110) and/or auxiliary (120) command signals; and **characterized in that** said verification block (23) comprising a time correction block (23d) to extend the time length of the first portion (100a) of said cardiac signal (100) if storage of said cardiac signal (100) into said storage unit (20) is active when said main (110) or auxiliary (120) command signal is received.

2. An apparatus as claimed in claim 1, **characterised in that** said main reference parameters (31) are representative of cardiac pathologies.

3. An apparatus as claimed in claim 1 or 2, **characterised in that** said cardiac signal (100) is representative of an electric activity of the patient's heart during operation of same.

4. An apparatus as claimed in claim 1, **characterised in that** said sensor means (10) comprises a predetermined number of electrodes (11) connectable to the patient (2) to detect said cardiac signal (100), said electrodes (11) being preferably in contact with the patient's skin when the apparatus (1) is in its use conditions.

5. An apparatus as claimed in claim 1, **characterised in that** said storage unite (20) comprises:
- a permanent memory (21) to store said cardiac signal (100);
- a time storage block (22) to store data representative of the time instants at which said cardiac signal (100) is detected and/or stored.

6. An apparatus as claimed in one or more of the preceding claims, **characterised in that** it further comprises a temporary storage register (50) connected to said sensor means (10) to temporarily store portions of said cardiac signal (100), which portions have a predetermined time duration.

7. An apparatus as claimed in claim 6, **characterised in that** said storage unit (20) is connected to said temporary register (50) to at least partly store the contents of same into said permanent memory (21) when said storage unit (20) receives the main command signal (110) from said comparator block (40).

8. An apparatus as claimed in claim 6 or 7, **characterised in that** said temporary register (50) has an initial address (51) and a final address (52) representative of the limits of said register (50), said cardiac signal (100) being sequentially stored into said register (50) from the initial address (51) to the final address (52), said cardiac signal (100) being overwritten in said register (50) starting from said initial address (51), when said final address (52) is reached in said sequential storage.

9. An apparatus as claimed in claim 1, **characterised in that** it further comprises a learning unit (60) provided with:
- a learning register (619 to store at least one initial portion (101) of said cardiac signal (100);
- processing means (62) to calculate input parameters (130) depending on characteristics of said initial portion (101) of the cardiac signal (100).

10. An apparatus as claimed in claim 9, **characterised in that** said learning unit (60) further comprises a calculation block (63) to calculate said main reference parameters (31) depending on said input parameters (130).

11. An apparatus as claimed in claim 10, **characterised in that** said learning unit (60) further comprises updating means (64) connected to said learning register (61) to store auxiliary portions (102) of said cardiac signal (100) at predetermined time intervals, said updating means (64) being further connected to said processing means (62) to activate the latter and update said input parameters (130) depending on characteristics of said auxiliary portions (102) of the cardiac signal (100).

12. An apparatus as claimed in claim 10 or 11, **characterised in that** said learning unit (60) further comprises an auxiliary register (65) holding general parameters (140) representative of cardiac pathologies, said calculation block (63) being connected to said auxiliary register (65) and processing means (62) to define said main reference parameters (31) depending on said general parameters (140) and input parameters (130).

13. An apparatus as claimed in claim 1, **characterised in that** said verification block (23) comprises:
- control means (23a) to verify whether storage of said cardiac signal (100) is active when one of said main (110) or auxiliary (120) command signals is received;
- timing means (23b) to store data representative of the time instant at which said main (110) or auxiliary (120) command signal is received;
- drive means (23c) to activate storage of said cardiac signal (100) if said storage were not active when said main (110) or auxiliary (120) command signal is received.

14. An apparatus as claimed in anyone of the preceding claims, **characterised in that** said storage unit (20) on reception of said main (110) or auxiliary (120) command signal, stores at least one first portion (100a) of said cardiac signal (100) having a predetermined time duration and detected after reception of said main (110) or auxiliary (120) command signal.

15. An apparatus as claimed in anyone of the preceding claims, **characterised in that** said storage unit (20) on reception of said main (110) or auxiliary (120) command signal, stores a second portion (100b) of said cardiac signal (100) detected before reception of said main (110) or auxiliary (120) command signal.

16. An apparatus as claimed in claim 15, **characterised in that** said second portion (100b) of the cardiac signal (100) is fetched from said temporary register (50) and stored into the permanent memory (21) of said storage unit (20).

17. An apparatus as claimed in claim 1, **characterised in that** it further comprises auxiliary-data input means (71) to enable the patient (2) to enter auxiliary data (150) relating to symptoms felt on activation of said driving block (70), said auxiliary data (150) being stored into a secondary register (24) of said storage unit (20).

18. An apparatus as claimed in anyone of the preceding claims, **characterised in that** it further comprises auxiliary activation means (81) to activate permanent storage of secondary portions (104) of said cardiac signal (100) at predetermined time intervals, irrespective of said main (110) or auxiliary (120) command signals.

19. An apparatus as claimed in anyone of the preceding claims, **characterised in that** it further comprises a communication interface (80) for a connection, preferably of the telephone or telecommunication type, with a remote computer (90) which is set for at least reception of the stored portions of said cardiac signal (100).

## Patentansprüche

1. Vorrichtung zur Detektion von Herzsignalen, umfassend:
- Fühlermittel (10), um mindestens ein Herzsignal (100) eines Patienten (2) zu detektieren;
- eine Speichereinheit (20), die mit den Fühlermitteln (10) verbunden ist, um mindestens teilweise das Herzsignal (100) zu speichern;
- einen Hilfsspeicher (30), der einen oder mehrere Hauptbezugsparameter (31) für das Herzsignal (100) enthält;
- einen Vergleichsblock (40), um das Herzsignal (100) und das eine oder die mehreren Hauptbezugsparameter (31) zu vergleichen und die Speichereinheit (20) in Abhängigkeit des Vergleichs über ein entsprechendes Hauptsteuersignal (110) zu aktivieren;
- einen Betätigungsblock (70), der durch den Patienten (2) aktivierbar ist, um die Speichereinheit (20) über das Hilfssteuersignal (120) zu aktivieren; wobei die Speichereinheit (20) einen Prüfblock (23) umfasst, der mit dem Betätigungsblock (70) und mit dem Vergleichsblock (40) verbunden ist, um das Hauptsteuersignal (110) und das Hilfssteuersignal (120) zu empfangen und einen Speicher des Herzsignals (100) in Abhängigkeit dieser Haupt- (100) und/oder Hilfs- (120) Steuersignalen zu aktivieren; und **dadurch gekennzeichnet, dass** der Prüfbock (23) einen Zeitkorrektionsblock (23d) umfasst, um die Zeitdauer des ersten Abschnittes (100a) des Herzsignals (100) zu verlängern, wenn die Speicherung des Herzsignals (100) in der Speichereinheit (20) aktiv ist, sobald das Hauptsteuersignal (110) oder Hilfssteuersignal (120) empfangen wird.

2. Vorrichtung nach Anspruch 1, **dadurch gekennzeichnet, dass** die Hauptbezugsparameter (31) für Herzpathologien repräsentativ sind.

3. Vorrichtung nach Anspruch 1 oder 2, **dadurch gekennzeichnet, dass** das Herzsignal (100) für eine elektrische Aktivität des Patientenherzens während dessen Funktion repräsentativ ist.

4. Vorrichtung nach Anspruch 1, **dadurch gekennzeichnet, dass** die Fühlermittel (10) eine vorgegebene Anzahl von Elektroden (11) umfassen, die mit dem Patienten (2) verbunden werden können, um das Herzsignal (100) zu detektieren, wobei die Elektroden (11) bevorzugter Weise mit der Haut des Patienten in Kontakt ist, sobald sich die Vorrichtung (1) unter Benutzungsbedingungen befindet.

5. Vorrichtung nach Anspruch 1, **dadurch gekennzeichnet, dass** die Speichereinheit (20) umfasst:
- einen Dauerspeicher (21), um das Herzsignal (100) zu speichern;
- einen Zeitspeicherblock (22), um die repräsentativen Daten der Zeitaugenblicke zu speichern, an denen das Herzsignal (100) detektiert und/oder gespeichert wurde.

6. Vorrichtung nach einem beliebigen der vorstehenden Ansprüche, **dadurch gekennzeichnet, dass** es überdies ein vorläufiges Speicherregister (50) umfasst, das mit den Fühlermitteln (10) verbunden ist, um die Abschnitte des Herzsignals (100) mit einer vorgegebenen Zeitdauer zu aktivieren.

7. Vorrichtung nach Anspruch 6, **dadurch gekennzeichnet, dass** die Speichereinheit (20) mit dem einstweiligen Register (50) verbunden ist, um mindestens teilweise den Inhalt desselben im Dauerspeicher (21) zu speichern, sobald die Speichereinheit (20) das Hauptsteuersignal (110) vom Vergleichsblock (40) erhält.

8. Vorrichtung nach Anspruch 6 oder 7, **dadurch gekennzeichnet, dass** das einstweilige Register (50) eine Anfangsadresse (51) und eine Endadresse (52) aufweist, die für die Registergrenzen (50) repräsentativ sind, wobei das Herzsignal (100) in der Reihenfolge im Register (50) von der Anfangsadresse (51) bis zur Endadresse (52) gespeichert wird, wobei das Herzsignal (100) im Register (50) ausgehend von der Anfangsadresse (51) überschrieben wird, sobald die Endadresse (52) bei dieser Speicherung in Reihenfolge erreicht wird.

9. Vorrichtung nach Anspruch 1, **dadurch gekennzeichnet, dass** sie überdies eine Lerneinheit (60)umfasst, die versehen ist mit:
- einem Lernregister (61), um mindestens einen Anfangsabschnitt (101) des Herzsignals (100) zu speichern;
- Verarbeitungsmittel (62), um die Einstellparameter (130) in Abhängigkeit der Merkmale des Anfangsabschnittes (101) des Herzsignals (100) zu berechnen.

10. Vorrichtung nach Anspruch 9, **dadurch gekennzeichnet, dass** die Lerneinheit (60) überdies einen Rechenblock (63) umfasst, um die Hauptbezugsparameter (31) in Abhängigkeit der Einstellparameter (130) zu berechnen.

11. Vorrichtung nach Anspruch 10, **dadurch gekennzeichnet, dass** die Lerneinheit (60) überdies Aktualisierungsmittel (64) umfasst, die mit dem Lernregister (61) verbunden sind, um in vorgegebenen Zeitabschnitten Hilfsabschnitte (102) des Herzsignals (100) zu speichern, wobei die Aktualisierungsmittel (64) überdies mit den Verarbeitungsmitteln (62) verbunden sind, um diese letzteren zu aktivieren und die Einstellparameter (130) in Abhängigkeit von Merkmalen der Hilfsabschnitte (102) des Herzsignals (100) zu aktualisieren.

12. Vorrichtung nach Anspruch 10 oder 11, **dadurch gekennzeichnet, dass** die Lerneinheit (60) überdies ein Hilfsregister (65) umfasst, das allgemeine Parameter (140) enthält, die für Herzpathologien repräsentativ sind, wobei der Rechenblock (63) mit dem Hilfsregister (65) und mit den Verarbeitungsmitteln (62) verbunden ist, um die Hauptbezugsparameter (31) in Abhängigkeit der allgemeinen Parameter (140) und der Einstellparameter (130) festzulegen.

13. Vorrichtung nach Anspruch 1, **dadurch gekennzeichnet, dass** der Prüfblock (23) umfasst:
- Prüfmittel (23a), um zu überprüfen, ob eine Speicherung des Herzsignals (100) aktiv ist, sobald eines der Hauptsteuersignale (110) oder Hilfssteuersignal (120) empfangen wird;
- Timermittel (23b), um die repräsentativen Daten des Zeitaugenblicks zu speichern, an denen das Hauptsteuersignal (110) oder Hilfssteuersignal (120) empfangen wird;
- Steuermittel (23c), um den Speicher des Herzsignals (100) zu aktivieren, sobald die Speicherung nicht aktiv ist, wenn das Hauptsteuersignal (110) oder Hilfssteuersignal (120) empfangen wird.

14. Vorrichtung nach einem beliebigen der vorstehenden Ansprüche, **dadurch gekennzeichnet, dass** die Speichereinheit (20), wenn sie das Hauptsteuersignal (110) oder Hilfssteuersignal (120) empfängt, mindestens einen ersten Abschnitt (100a) des Herzsignals (100) mit einer vorgegebenen Zeitdauer speichert, das nach dem Empfang des Hauptsteuersignals (110) oder Hilfssteuersignals (120) detektiert wurde.

15. Vorrichtung nach einem beliebigen der vorstehenden Ansprüche, **dadurch gekennzeichnet, dass** die Speichereinheit (20) sobald sie das Hauptsteuersignal (110) oder Hilfssteuersignal (120) empfängt, einen zweiten Abschnitt (100b) des Herzsignals (100) speichert, das vor dem Empfang des Hauptsteuersignals (110) oder Hilfssteuersignals (120) detektiert wurde.

16. Vorrichtung nach Anspruch 15, **dadurch gekennzeichnet, dass** der zweite Abschnitt (100b) des Herzsignals (100) vom einstweiligen Register (50) abgenommen und im Dauerspeicher (21) der Speichereinheit (20) gespeichert wird.

17. Vorrichtung nach Anspruch 1, **dadurch gekennzeichnet, dass** sie überdies Hilfsdateneingabemittel (71) umfasst, um dem Patienten (2) zu erlauben, Hilfsdaten (150) bezüglich von im Bereich der Aktivierung des Betätigungsblocks (70) wahrgenommenen Symptomen einzugeben, wobei die Hilfsdaten (150) in einem Nebenregister (24) der Speichereinheit (20) gespeichert sind.

18. Vorrichtung nach einem beliebigen der vorstehenden Ansprüche, **dadurch gekennzeichnet, dass** sie überdies Hilfsaktivierungsmittel (81) umfasst, um eine Dauerspeicherung von Nebenabschnitten (104) des Herzsignals (100) in vorgegebenen Zeitabschnitten unabhängig von den Hauptsteuersignalen (110) oder Hilfssteuersignalen (120) zu aktivieren.

19. Vorrichtung nach einem beliebigen der vorstehenden Ansprüche, **dadurch gekennzeichnet, dass** sie überdies eine Übertragungsschnittstelle (80) für eine Verbindung, bevorzugter Weise telefonischer oder telematischer Art mit einem abgelegenen Rechner (90) umfasst, wobei dieser letztere für mindestens den Empfang der gespeicherten Abschnitte des Herzsignals (100) bereitgestellt ist.

## Revendications

1. Appareil pour détecter des signaux cardiaques, comprenant:
- des moyens capteurs (10) pour détecter au moins un signal cardiaque (100) d'un patient (2);
- une unité de stockage (20) reliée auxdits moyens capteurs (10) pour mémoriser au moins partiellement ledit signal cardiaque (100);
- une mémoire auxiliaire (30) contenant un ou plusieurs paramètres de référence (31) pour ledit signal cardiaque (100);
- un bloc comparateur (40) pour comparer réciproquement ledit signal cardiaque (100) et lesdits un ou plusieurs paramètres de référence (31) et activer ladite unité de stockage (20) en fonction de ladite comparaison par l'intermédiaire d'un signal de commande principal (110) y relatif;
- un bloc d'actionnement (70) qui doit être commandé par le patient (2) pour activer ladite, unité de stockage (20) par l'intermédiaire d'un signal de commande auxiliaire (120);
où ladite unité de stockage (20) comprend un bloc de vérification (23) relié audit bloc d'actionnement (70) et audit bloc comparateur pour recevoir ledit signal de commande principal (110) et ledit signal de commande auxiliaire (120) et activer la mémorisation dudit signal cardiaque (100) en fonction desdits signaux de commande principal (110) et/ou auxiliaire (120); et
**caractérisé en ce que** ledit bloc de vérification (23) comprend un bloc de correction dans le temps (23d) pour prolonger la longueur temporelle de la première portion (100a) dudit signal cardiaque (100) si la mémorisation dudit signal cardiaque (100) dans ladite unité de stockage (20) est active quand ledit signal de commande principal (110) ou auxiliaire (120) est reçu.

2. Appareil selon la revendication 1, **caractérisé en ce que** lesdits paramètres de référence principaux (31) sont représentatifs de pathologies cardiaques.

3. Appareil selon la revendication 1 ou 2, **caractérisé en ce que** ledit signal cardiaque (100) est représentatif d'une activité électrique du coeur du patient pendant son fonctionnement.

4. Appareil selon la revendication 1, **caractérisé en ce que** lesdits moyens capteurs (10) comprennent un nombre prédéterminé d'électrodes (11) qui peuvent être reliées au patient (2) pour détecter ledit signal cardiaque (100), lesdites électrodes (11) étant de préférence en contact avec la peau du patient quand l'appareil (1) est en cours d'utilisation.

5. Appareil selon la revendication 1, **caractérisé en ce que** ladite unité de stockage (20) comprend:
- une mémoire permanente (21) pour mémoriser ledit signal cardiaque (100);
- un bloc de mémorisation dans le temps (22) pour mémoriser les données représentatives des instants de temps dans lesquels ledit signal cardiaque (100) est détecté et/ou mémorisé.

6. Appareil selon une ou plusieurs des revendications précédentes, **caractérisé en ce qu'**il comprend en outre un registre de mémoire intermédiaire (50) relié auxdits moyens capteurs (10) pour stocker des portions dudit signal cardiaque (100), ces portions ayant une durée de temps prédéterminée.

7. Appareil selon la revendication 6, **caractérisé en ce que** ladite unité de stockage (20) est reliée audit registre intermédiaire (50) pour stocker au moins partiellement le contenu de celui-ci dans ladite mémoire permanente (21) quand ladite unité de stockage (20) reçoit le signal de commande principal (110) dudit bloc comparateur (40).

8. Appareil selon la revendication 6 ou 7, **caractérisé en ce que** ledit registre intermédiaire (50) a une adresse initiale (51) et une adresse finale (52) représentatives des limites dudit registre (50), ledit signal cardiaque (100) étant stocké en séquence dans ledit registre (50) depuis l'adresse initiale (51) jusqu'à l'adresse finale (52), ledit signal cardiaque (100) étant recouvert dans ledit registre (50) à partir de ladite adresse initiale (51), quand ladite adresse finale (52) est reçue dans ladite mémoire séquentielle.

9. Appareil selon la revendication 1, **caractérisé en ce qu'**il comprend en outre une unité d'apprentissage (60) munie de:
- un registre d'apprentissage (61) pour stocker au moins une portion initiale (101) dudit signal cardiaque (100);
- des moyens de traitement (62) pour calculer les paramètres d'entrée (130) en fonction des caractéristiques de ladite portion initiale (101) du signal cardiaque (100).

10. Appareil selon la revendication 9, **caractérisé en ce que** ladite unité d'apprentissage (60) comprend en outre un bloc de calcul (63) pour calculer lesdits paramètres de référence principaux (31) en fonction desdits paramètres d'entrée (130).

11. Appareil selon la revendication 10, **caractérisé en ce que** ladite unité d'apprentissage (60) comprend en outre des moyens de remise à jour (64) reliés audit registre d'apprentissage (61) pour stocker les portions auxiliaires (102) dudit signal cardiaque (100) à intervalles de temps prédéterminés, lesdits moyens de remise à jour (64) étant en outre reliés auxdits moyens de traitement (62) pour activer ces derniers et remettre à jour lesdits paramètres d'entrée (130) en fonction des caractéristiques desdites portions auxiliaires (102) du signal cardiaque (100).

12. Appareil selon la revendication 10 ou 11, **caractérisé en ce que** ladite unité d'apprentissage (60) comprend en outre un registre auxiliaire (65) contenant des paramètres généraux (140) représentatifs des pathologies cardiaques, ledit bloc de calcul (63) étant relié audit registre auxiliaire (65) et auxdits moyens de traitement (62) pour définir lesdits paramètres de référence principaux (31) en fonction desdits paramètres généraux (140) et desdits paramètres d'entrée (130).

13. Appareil selon la revendication 1, **caractérisé en ce que** ledit bloc de vérification (23) comprend:
- des moyens de contrôle (23a) pour vérifier si la mémorisation dudit signal cardiaque (100) est active quand on reçoit un desdits signaux de commande principal (110) ou auxiliaire (120);
- des moyens temporisateurs (23b) pour mémoriser les données représentatives de l'instant de temps auquel on reçoit ledit signal de commande principal (110) ou auxiliaire (120);
- des moyens de commande (23c) pour activer la mémorisation dudit signal cardiaque (100) si ladite mémorisation n'était pas active quand on reçoit ledit signal de commande principal (110) ou auxiliaire (120).

14. Appareil selon l'une quelconque des revendications précédentes, **caractérisé en ce que** ladite unité de mémoire (20) lors de la réception dudit signal de commande principal (110) ou auxiliaire (120), mémorise au moins une première portion (100a) dudit signal cardiaque (100) ayant une durée de temps prédéterminée et détectée après réception dudit signal de commande principal (110) ou auxiliaire (120).

15. Appareil selon l'une quelconque des revendications précédentes, **caractérisé en ce que** ladite unité de mémoire (20) lors de la réception dudit signal de commande principal (110) ou auxiliaire (120), mémorise une deuxième portion (100b) dudit signal cardiaque (100) détectée avant la réception dudit signal de commande principal (110) ou auxiliaire (120).

16. Appareil selon la revendication 15, **caractérisé en ce que** ladite deuxième portion (100b) du signal cardiaque (100) est prélevée dudit registre intermédiaire (50) et stockée dans la mémoire permanente (21) de ladite unité de mémoire (20).

17. Appareil selon la revendication 1, **caractérisé en ce qu'**il comprend en outre des moyens d'introduction de données auxiliaires (71) pour permettre au patient (2) d'introduire des données auxiliaires (150) concernant des symptômes perçus lors de l'activation dudit bloc d'actionnement (70), lesdites données auxiliaires (150) étant stockées dans un registre secondaire (24) de ladite unité de mémoire (20).

18. Appareil selon l'une quelconque des revendications précédentes, **caractérisé en ce qu'**il comprend en outre des moyens d'activation auxiliaires (104) dudit signal cardiaque (100) à intervalles de temps prédéterminés, sans tenir compte desdits signaux de commande principal (110) ou auxiliaire (120).

19. Appareil selon l'une quelconque des revendications précédentes, **caractérisé en ce qu'**il comprend en outre une interface de communication (80) pour une connexion, de préférence de type téléphonique ou télématique, avec un ordinateur à distance (90), ce dernier étant prévu au moins pour la réception des portions mémorisées dudit signal cardiaque (100).
